# EUROPÄISCHE PATENTSCHRIFT

(11) **EP 2 510 878 B1**
(45) Veröffentlichungstag und Bekanntmachung des Hinweises auf die Patenterteilung: **26.02.2014**
(21) Anmeldenummer: 11162043.1
(22) Anmeldetag: 12.04.2011
(51) Int. Cl.: A61B 6/00, A61B 6/12, A61B 6/14, G06T 11/00, G06T 3/40

(54) **Verfahren zur Generierung einer radiologischen dreidimensionalen digitalen Volumentomographie-Aufnahme eines Patienten-Körperteils**
Method for generating a radiological three dimensional digital volume tomography image of part of a patient's body
Procédé de génération d'un enregistrement de tomographie volumétrique numérique tridimensionnelle radiologique d'une partie du corps d'un patient

(43) Veröffentlichungstag der Anmeldung: 17.10.2012
(73) Patentinhaber: Abboud, Marcus, 53721 Siegburg (DE)
(72) Erfinder: Abboud, Marcus, 53721 Siegburg (DE)
(74) Vertreter: Eberlein, Jasper

(56) Entgegenhaltungen:
- WO-A1-02/32307
- WO-A1-99/44503
- WO-A2-01/33511
- WO-A2-2004/100767
- WO-A2-2006/094156
- GB-A- 2 449 113
- US-A1- 2003 086 535
- US-A1- 2007 122 020
- US-A1- 2009 018 437
- US-A1- 2009 086 887
- US-A1- 2010 278 311

## Beschreibung

Die Erfindung bezieht sich auf ein Verfahren zur Generierung einer radiologischen dreidimensionalen digitalen Volumentomographie-Aufnahme eines Patienten-Körperteils, insbesondere eines Patientenkopfes, und hierbei besonders bevorzugt eines Patientenkiefers. Als zahnärztliches und hals-nasen-ohrenärztliches bildgebendes radiologisches Tomographie-Verfahren wird häufig die digitale Volumentomographie eingesetzt. Bei der digitalen Volumentomographie wird ein kegelförmiger Röntgenstrahl auf einen in einem konstanten Abstand gegenüberstehenden zweidimensionalen Detektor gerichtet. Die Anordnung, bestehend aus der Röntgenstrahlungsquelle und dem Detektor, wird während der Aufnahme um den Patientenkopf herum bewegt, wobei für eine Aufnahme 10-30 s benötigt werden.

Die digitale Volumentomographie hat, im Vergleich zu anderen dreidimensionalen bildgebenden radiologischen Verfahren, den Vorteil einer relativ geringen Strahlenbelastung für den Patienten. Allerdings ist die Qualität der hieraus gewonnenen und errechneten dreidimensionalen Bilder des Patientenkopfes stark schwankend, und ist nur schwer einzuschätzen. Die Gründe für die schwankende Bildqualität sind zum Einen prinzipbedingt und physikalischer Natur, und liegen zum Anderen in der langen Aufnahme-Dauer, wegen der unerwünschte Eigenbewegungen des Patientenkopfes, der Röntgenstrahlungsquelle und des Detektors während der Aufnahme nicht ausgeschlossen werden können. Durch die Eigenbewegungen wird die Qualität der Bilder erheblich verschlechtert. In der Praxis werden in dem dreidimensionalen Bild die Größenverhältnisse und -relationen häufig mit einem Fehler von bis zu mehreren Millimetern dargestellt, Dies ist zwar in der Diagnostik nicht in jedem Fall problematisch, jedoch wird die digitale Volumentomographie insbesondere in der Zahnheilkunde zur Planung von dentalen Implantaten im Patientenkiefer eingesetzt. Hierbei ist eine hohe geometrische Genauigkeit erforderlich, um eine hohe Qualität der Implantat-Versorgung sicherstellen zu können.

Aus WO 02/32307 A1 ist ein bildgebendes Verfahren bekannt, bei dem ein Katheter an der Katheterspitze 2 radio-opake Marker aufweist, mit deren Hilfe eine in einem Röntgenbild zu bestimmende Distanz skaliert wird.

US 2009/0086887 A1 befasst sich mit der Korrektur und Normierung radiologischer Bilder anhand der Größe der radiologischen Bilder.

US 2007/0122020 A1 offenbart ein Verfahren zur Kalibrierung eines Gerätes zur Erfass ung dreidimensionaler radiologischer Aufnahmen.

In US 2009/0018437 A1 wird ein mehrstufiges bildgebendes Verfahren beschrieben, mit dem Parrallaxe- und Distorsions-Fehler korrigiert werden können.

WO 01/33511 A2 offenbart ein dreidimensionales Computertomographie-Verfahren, bei dem zur Referenzierung ein Rahmen eingesetzt wird.

US 2010/0278311 A1 beschreibt ein zweidimensionales bildgebendes Verfahren, bei dem ein großflächiges radio-opakes Referenzelement eingesetzt wird.

Aufgabe der Erfindung ist es demgegenüber, ein Verfahren zur Generierung einer radiologischen dreidimensionalen Aufnahme, insbesondere einer digitalen Volumentomographie-Aufnahme, eines Patienten-Körperteils mit der Möglichkeit einer Qualitätskontrolle und/oder einer verbesserten Genauigkeit zu schaffen.

Diese Aufgabe wird erfindungsgemäß gelöst durch die Merkmale des Patentanspruches 1.

Gemäß dem Verfahren nach Anspruch 1 ist vorgesehen, dass vor der Anfertigung der Aufnahme ein Markerträger an dem Patienten-Körperteil, und bevorzugt in dem Patientenmund fixiert wird. Beispielsweise kann der Markerträger ein von dem Patientenkiefer abgeformtes Individualformteil aufweisen, mit dem der Markerträger formschlüssig und unverrückbar auf den Patientenkiefer aufgesetzt werden kann. Die Fixierung kann durch den Aufbiss des Patienten erfolgen.

Der Markerträger weist einen nicht-radioopaken Trägerkörper auf, der praktisch keine Röntgenstrahlung absorbiert und daher für ein Röntgengerät weitgehend unsichtbar ist. An oder in dem Trägerkörper sind mehrere radioopake Markerelemente fixiert, deren Ist-Größe S und/oder Ist-Abstand D zueinander bekannt ist.

Zunächst wird mit dem digitalen Volumentomographie-Gerät eine Aufnahme des betreffenden Körperteils, beispielsweise des Patientenkiefers, zur Erzeugung eines dreidimensionalen Rohbildes des Körperteils gemacht, wie dies aus dem Stand der Technik bereits bekannt ist. Anschließend wird in dem Rohbild bezüglich einiger oder aller Markerelemente ihre Größe S' und/oder die Abstände D' der Markerelemente zueinander vermessen.

Die vermessenen Werte für die Markerelement-Größe S' bzw. die Markerelement-Abstände D' werden mit den jeweiligen Ist-Werten S, D verglichen, und hieraus ein Abgleichungswert QS, QD erzeugt. Dieser Abgleichungswert QS, QD kann beispielsweise jeweils der Quotient aus dem jeweiligen Ist-Wert S, D und den vermessenen Werten S', D' sein.

Anschließend wird das Rohbild anhand der gewonnenen Abgleichungswerte QS, QD korrigiert. Das Rohbild kann beispielsweise normiert werden, indem es um den Wert des Quotienten aus dem Ist-Wert und dem gemessenen Wert gestreckt oder gestaucht wird. Das auf diese Weise gewonnene korrigierte dreidimensionale Bild des Körperteils bzw. des Patientenkiefers ist insbesondere bezüglich der Größenverhältnisse und Abstände erheblich genauer als das Rohbild. Insbesondere für die Planung von dentalen Implantaten wird auf diese Weise eine exakte dreidimensionale Abbildung des Patientenkiefers zur Verfügung gestellt, die die Basis für die anschließende genaue Planung und Realisierung der implantologischen dentalen Versorgung darstellt.

Vorzugsweise ist die Korrektur eine Größenkorrektur des Rohbildes anhand der Rohbild-Abstände D' im Verhältnis zu den Ist-Abständen D der Markerelemente zueinander. Aus dem Quotienten der Rohbild-Abstände D' und der Ist-Abstände D der Markerelemente zueinander kann unmittelbar ein Korrekturwert generiert werden, um den das Rohbild zu korrigieren ist.

Alternativ oder ergänzend hierzu kann ein Qualitätsindikator ausgegeben werden, dessen Wert sich aus der Abweichung der Rohbild-Größe S' im Verhältnis zu der Ist-Größe S der Markerelemente ergibt. Als Basis für den Qualitätsindikator kann also auch hier ein Quotient benutzt werden. Der Größen-Qualitätsindikator gibt Auskunft darüber, von welcher Qualität das Rohbild an dem Ort des jeweiligen Markerelementes ist. Bei Verwacklungen oder Unschärfen in dem Rohbild wird das Markerelement in dem Rohbild beispielsweise erheblich größer dargestellt, als es tatsächlich ist. Zwar kann mit Hilfe des Qualitätsindikators keine unmittelbare Korrektur des Rohbildes durchgeführt werden, jedoch gibt er dem Nutzer des Rohbildes eine Auskunft darüber, inwieweit der Nutzer dem Rohbild vertrauen kann.

Gemäß einer bevorzugten Ausgestaltung sind mindestens 10 Markerelemente vorgesehen, wobei jedes Markerelement zu seinen Nachbar-Markerelementen jeweils in einem konstanten Ist-Abstand von höchstens 3,0 cm angeordnet ist. In bzw. an dem Markerträger ist also ein Netz von Markerelementen aufgespannt, dessen Maschenweite über das gesamte Netz stets gleich ist, und dessen Maschenweite höchstens 3,0 cm beträgt. Hierdurch wird die Möglichkeit eröffnet, die Größenkorrektur anhand der Rohbild-Abstände D' im Verhältnis zu den Ist-Abstanden D in dem Rohbild an mehreren Orten lokal vorzunehmen. Hierdurch wird der Tatsache Rechnung getragen, dass die Verzerrungen in dem Rohbild tatsächlich lokal verschieden ausfallen können.

Die Markerelemente können jeweils flächig oder im Raum verteilt und auf rechtwinklig zueinander stehenden Linien angeordnet sein. Besonders bevorzugt bilden drei zueinander benachbarte Markerelemente jedoch ein gleichseitiges Dreieck. Bei dieser Anordnung kann mit relativ wenigen Markerelementen, die grundsätzlich wegen ihrer Radioopazität selbst auch Quelle von Artefakten sein können, ein Netz mit einer ausreichenden Dichte an Markerelementen in dem relevanten Raum aufgespannt werden.

Gemäß einer bevorzugten Ausführungsform sind die Markerelemente in mindestens zwei Querebenen angeordnet, also in zwei Horizontalebenen. Hierdurch wird sichergestellt, dass die Größenkorrektur des Rohbildes anhand der Abstände D, D' der Markerelemente zueinander in Bezug auf alle drei Raumachsen vorgenommen werden kann.

Vorzugsweise ist die Radioopazität der Markerelemente verschieden. Da die Markerelemente wegen ihrer Radioopazität bezüglich der Röntgenstrahlung auch reflexive Eigenschaften haben, die vom Grad ihrer Radioopazität abhängig sind, können beispielsweise durch Verringerung der Radioopazität der Markerelemente in besonders sensiblen Bereichen Artefakte in diesen Bereichen verringert oder vermieden werden.

Ferner ist vorgesehen, eine Kalibrierung der Helligkeit des Rohbildes auf Grundlage der bekannten verschiedenen Radioopazitäts-Werte der Markerelemente vorzunehmen. Beispielsweise können die Radioopazitäts-Werte der Markerelemente drei bis sechs verschiedene Werte haben, so dass annähernd das gesamte Radioopazitäts-Spektrum des aufzunehmenden Körperteils abgedeckt wird. Es wird auf diese Weise in dem Rohbild ein objektiver Maßstab für die Radioopazität zur Verfügung gestellt. Mit diesem Maßstab kann das Rohbild automatisch kalibriert werden, d.h. die in dem Rohbild abgebildeten Strukturen des Körperteils können bezüglich ihrer Radioopazität objektiv eingeordnet werde. Dies erleichtert die Interpretation des dreidimensionalen Bildes. Insbesondere können Knochen und Gewebe einfacherer und sicherer voneinander unterschieden werden, was bei der zahnärztlichen Implantologie von essentieller Bedeutung ist. Der Markerträger kann mehrere über den Körperteil bzw. den Bildraum verteilte Radioopazitäts-Spektren aufweisen, die jeweils aus mehreren Markerelementen verschiedener Radioopazität bestehen, so dass die Kalibrierung lokal vorgenommen werden kann. Hiermit kann dem Umstand Rechnung getragen werden, dass die Abbildungsqualität bei der digitalen Volumentomographie prinzipbedingt lokal sehr verschieden ausfallen kann.

Vorzugsweise ist der Markerträger als intraoraler Markerträger ausgebildet, und sind mehrere Markerelemente innerhalb des Zahnbogens angeordnet. Besonders bevorzugt ist mindestens ein Markerelement außerhalb des Zahnbogens, und besonders bevorzugt außerhalb des Patientenmundes angeordnet. Die Anordnung außerhalb des Zahnbogens bzw. des Patientenmundes erlaubt die Erzeugung von Abgleichungswerten beispielsweise im Bereich des Kieferknochens des Patientenkiefers. Gerade in diesem Bereich ist für die Implantatplanung eine hohe Genauigkeit wünschenswert.

Im Folgenden werden unter Bezugnahme auf die Zeichnungen zwei Ausführungsbeispiel der Erfindung näher erläutert.

Es zeigen:
Figur 1 eine perspektivische Ansicht eines oralen Markerträgers,
Figur 2 einen Längsschnitt durch den Markerträger der Figur 1,
Figur 3 einen Querschnitt durch einen Patientenkiefer mit eingesetztem intraoralen Markerträger und einem digitalen Volumentomographie-Gerät, und
Figur 4 ein Ablaufdiagramm eines Verfahrens zur Generierung einer radiologischen dreidimensionalen Aufnahme eines Patientenkopfes.

Die in den Figuren dargestellten Ausführungsbeispiele beziehen sich auf eine zahnärztliche Anwendung, wobei der Körperteil vorliegend ein Patientenkiefer ist.

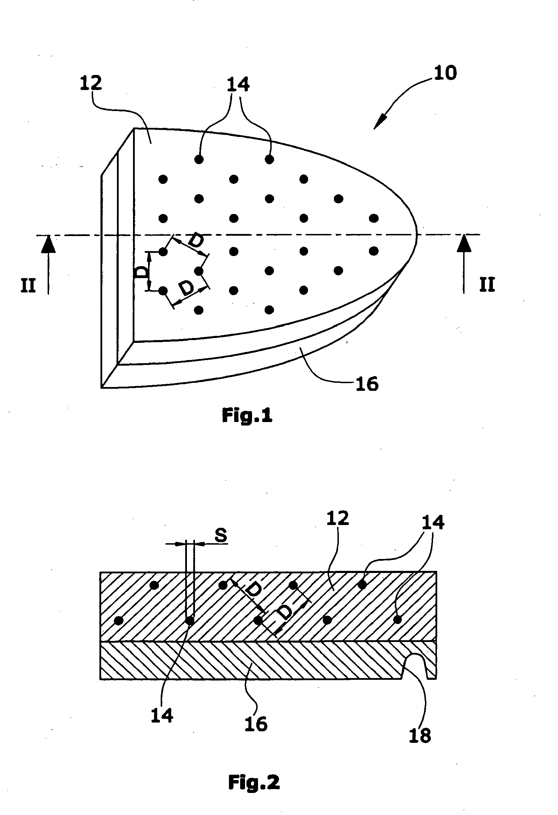

In den Figuren 1 und 2 ist schematisch ein intraoraler Markerträger 10 dargestellt, wie er zur Generierung einer radiologischen dreidimensionalen Aufnahme mit einem digitalen Volumentomographie-Gerät geeignet ist. Der Markerträger 10 besteht aus einem radiologisch nicht sichtbaren Trägerkörper 12 aus einem geeigneten Kunststoff. An dem Trägerkörper 12 ist ein Individualformteil 16 angebracht, beispielsweise verklebt, das Zahnausnehmungen 18 aufweist, die komplementär zu den Patientenzähnen 24 geformt sind. Mit dem Individualformteil 16, das eine Abformung des betreffenden Patientenkiefers 22,23 darstellt, wird ein fester, unverrückbarer und definierter Sitz des Markerträger 10 auf dem Patientenkiefer 22,23 in dem Patientenmund sichergestellt.

In den Trägerkörper 12 sind radiologisch sichtbare, also radioopake, Markerelemente 14 in zwei Ebenen liegend und gleichmäßig verteilt angeordnet. Drei zueinander benachbarte Markerelemente 14 bilden jeweils ein gleichseitiges Dreieck, und sind jeweils mit einem für alle Markerelemente 14 gleichen Ist-Abstand D zueinander beabstandet, der nicht größer als 3,0 cm ist. Alle Markerelemente 14 haben eine identische Größe S, die beispielsweise bei kugelförmigen Markerelementen 14 dem Durchmesser entspricht. Die Größe S beträgt maximal wenige Millimeter. In der Figur 2 ist ein zweites Ausführungsbeispiel eines komplexeren Markerträgers 20 dargestellt, der in den Patientenmund eingesetzt ist. Ferner ist ein um eine Vertikalachse rotierender Rotor 44 eines digitalen Volumentomographie-Gerätes dargestellt, der eine Röntgenstrahlungsquelle 40, die einen konusförmigen Röntgenstrahl generiert, und gegenüberliegend einen zweidimensionalen Detektor 42 trägt.

Der Markerträger 20 ist im Querschnitt H-förmig ausgebildet und weist einen horizontalen Trägerkörper-Teil 26 und an seinem Außenumfang jeweils nach oben und unten abragende vertikale Tragerkörper-Teile 30,32 auf. In den Grundebenen der Trägerkörper-Teile 26,30,32 sind jeweils die Markerelemente 14 flächig verteilt angeordnet. Auf der Oberseite und der Unterseite des horizontalen Trägerkörper-Teils ist jeweils ein Individualformteil 28,29 fixiert, das von dem Oberkiefer 22 bzw. dem Unterkiefer 23 abgeformt ist. Die Anordnung der Markerelemente 14 entspricht prinzipiell der in dem Markerträger 10 der Figuren 1 und 2.

Die meisten Markerträger 14 in dem horizontalen Trägerkörper-Teil 26 sind innerhalb der von den Patienten-Zähnen 24 des Patienten-Oberkiefers 22 und -Unterkiefers 23 aufgespannten Zahnbögen 36,37 angeordnet. Insbesondere die Markerelemente 14' in den vertikalen Trägerkörper-Teilen 30,32 sind außerhalb der Zahnbögen 36,37, jedoch innerhalb des Patientenmundes angeordnet.

Das Verfahren zur Generierung einer radiologischen dreidimensionalen Aufnahme des Patientenkiefers läuft wie folgt ab:
Zunächst wird in einem ersten Verfahrensschritt 50 der Markerträger 20 in den Patientenmund eingesetzt. Die Fixierung des Markerträger 20 in Patientenmund erfolgt durch den Aufbiss des Patienten.

Anschließend wird in den nächsten Verfahrensschritt 52 mit dem digitalen Volumentomographie-Gerät eine Aufnahme des Patientenkiefers zur Erzeugung eines dreidimensionalen Rohbildes des Patientenkopfes bzw. - kiefers gemacht. Daraufhin wird in einem weiteren Verfahrensschritt 54 in dem Rohbild bezüglich aller Markerelemente ihrer Größe S' und die Abstände D' der Markerelemente zueinander vermessen.

Daraufhin werden in dem folgenden Verfahrensschritt 56 die vermessenen Werte für die Markerelement-Abstände D' mit dem bekannten Ist-Wert D abgeglichen, bzw. hieraus ein Quotient generiert. Aus den lokalen Quotienten der Rohbild-Abstände D' und der konstanten Ist-Abstände D der Markerelemente zueinander wird unmittelbar ein Korrekturwert QD generiert, um den das Rohbild in einem anschließenden Verfahrensschritt 58 lokal zu korrigieren. Die Korrektur ist jeweils eine Größenkorrektur des Rohbildes anhand der Rohbild-Abstände D' im Verhältnis zu den Ist-Abeständen D der Markerelemente zueinander.

Das auf diese Weise gewonnene dreidimensionale korrigierte Bild des Patientenkopfes bzw. des Patientenkiefers ist insbesondere bezüglich der Größenverhältnisse und Abstände erheblich genauer als das Rohbild. Insbesondere für die Planung von dentalen Implantaten wird auf diese Weise eine exakte dreidimensionale Abbildung des Patientenkiefers zur Verfügung gestellt, die die Basis für die anschließende genaue Planung und Realisierung der implantologischen dentalen Versorgung darstellt.

Ergänzend hierzu werden mehrere lokale Qualitätsindikatoren QS ausgegeben bzw. in das korrigierte Rohbild eingeblendet, deren Wert sich aus der relativen Abweichung der Rohbild-Größe S' zu der Ist-Größe S der Markerelemente ergibt. Der Größen-Qualitätsindikator QS gibt Auskunft darüber, von welcher Qualität das Rohbild an dem betreffenden Ort ist, und gibt insbesondere Auskunft darüber, inwieweit dem Rohbild bzw. dem korrigierten Rohbild vertraut werden kann.

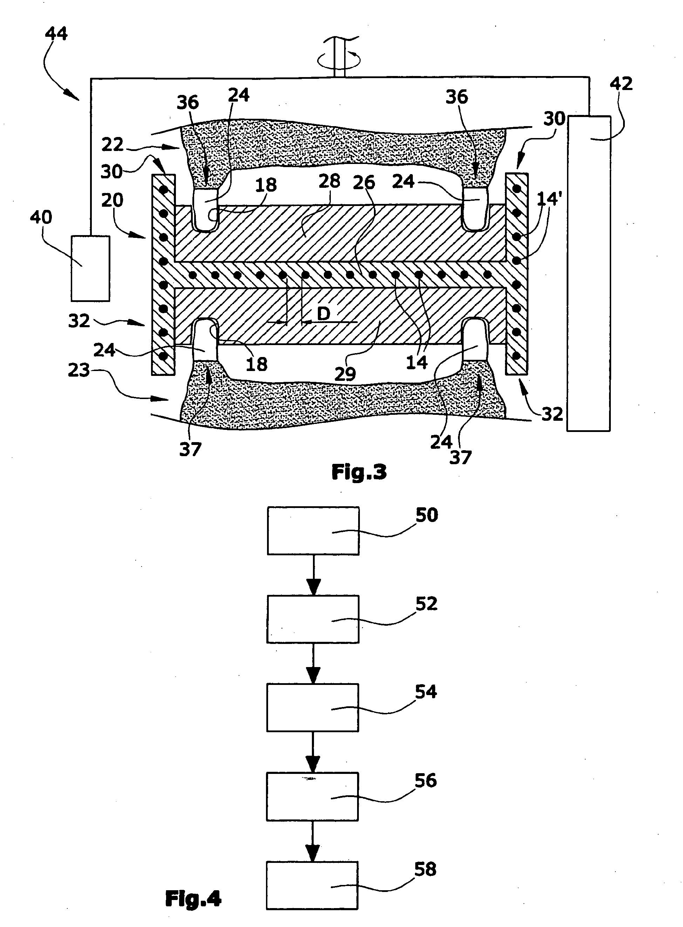

Die Radioopazität der Markerelemente 14,14' ist verschieden. Bezogen auf das Ausführungsbeispiel der Fig. 3 kann an dem vertikalen Trägerkörper-Teil 30 an vier Positionen des Zahnbogens ein Spektrum von drei bis sechs vertikal zueinander benachbarten Markerelementen 14' verschiedener Radioopazität vorgesehen sein. Diese werden in dem Rohbild dazu genutzt, das Rohbild bezüglich seiner Helligkeit und seines Kontrastes jeweils lokal zu kalibrieren. Diese Kalibrierung kann in den vier Bereichen durchaus verschieden ausfallen. Das hierbei gewonnene Bild erleichtert erheblich die Unterscheidung von Knochen und Gewebe.

## Patentansprüche

1. Verfahren zur Generierung einer radiologischen dreidimensionalen digitalen Volumentomographie-Aufnahme eines Patienten-Körperteils, insbesondere eines Patientenkopfes, mit den Verfahrensschritten:
Fixieren eines Markerträgers (10; 20) an dem Körperteil, bevorzugt in dem Patientenmund, wobei der Markerträger (10; 20) einen nicht-radioopaken Trägerkörper (12; 26) aufweist, an dem mehrere radioopake Markerelemente (14,14') fixiert sind, deren Ist-Größe S und/oder Ist-Abstand D zueinander bekannt ist,
Aufnahme des Körperteils mit einem digitalen Volumentomographie-Gerät zu Erzeugung eines dreidimensionalen Rohbildes des Körperteils,
Vermessen der Größe S' und/oder des Abstandes D' der Markerelemente (14) zueinander in dem Rohbild,
Erzeugung von Abweichungswerten QS, QD aus einem Abgleich der Rohbild-Größe S' und/oder der Rohbild-Abstände D' mit den jeweiligen Ist-Werten S, D, und
Korrektur des Rohbildes anhand der Abgleichungswerte QS, QD.

2. Verfahren zur Generierung einer dreidimensionalen Aufnahme eines Körperteils nach Anspruch 1, wobei die Korrektur eine Größenkorrektur des Rohbildes anhand der Rohbild-Abstände D' im Verhältnis zu den Ist-Abständen D der Markerelemente (14,14') ist.

3. Verfahren zur Generierung einer dreidimensionalen Aufnahme eines Körperteils nach einem der vorangegangenen Ansprüche, wobei ein Qualitätsindikator ausgegeben wird, dessen Wert sich aus der Abweichung der Rohbild-Größe S' zu der Ist-Größe S der Markerelemente (14,14') ergibt.

4. Verfahren zur Generierung einer dreidimensionalen Aufnahme eines Körperteils nach einem der vorangegangenen Ansprüche, wobei mindestens zehn Markerelemente (14,14') vorgesehen sind, wobei jedes Markerelement (14,14') zu seinen Nachbar-Markerelementen (14,14') jeweils in einem konstanten Ist-Abstand von höchstens 3,0 cm angeordnet ist, und wobei die Größenkorrekter mehrfach lokal anhand der lokalen Vergleichswerte vorgenommen wird.

5. Verfahren zur Generierung einer dreidimensionalen Aufnahme eines Körperteils nach einem der vorangegangenen Ansprüche, wobei drei zueinander benachbarte Markerelemente (14,14') jeweils ein gleichseitiges Dreieck bilden.

6. Verfahren zur Generierung einer dreidimensionalen Aufnahme eines Körperteils nach einem der vorangegangenen Ansprüche, wobei die Markerelemente (14) in mindestens zwei Querebenen angeordnet sind.

7. Verfahren zur Generierung einer dreidimensionalen Aufnahme eines Körperteils nach einem der vorangegangenen Ansprüche, wobei die Radioopazität der Markerelemente (14,14') verschieden ist, mit dem Verfahrensschritt:
Kalibrierung der Helligkeit des Rohbildes auf Grundlage der bekannten verschiedenen Radioopazitäts-Werte der Markerelemente (14,14').

8. Verfahren zur Generierung einer dreidimensionalen Aufnahme eines Körperteils nach einem der vorangegangenen Ansprüche , wobei der Körperteil der Patientenkiefer (22,23) ist, und mehrere Markerelemente (14) Innerhalb des Zahnbogens (36,37) angeordnet sind.

9. Verfahren zur Generierung einer dreidimensionalen Aufnahme eines Körperteils nach einem der vorangegangenen Ansprüche, wobei mindestens ein Markerelement (14') außerhalb des Zahnbogens (36,37) angeordnet Ist.

10. Verfahren zur Generierung einer dreidimensionalen Aufnahme eines Körperteils nach Anspruch 8 oder 9, wobei mindestens ein Markerelement außerhalb des Patientenmundes angeordnet ist.

## Claims

1. Method for generating a radiologic three-dimensional digital volume tomography image of a patient's body part, in particular of a patient's head, including the steps:
fixation of a marker support (10;20) to the body part, preferably in the patient's mouth, wherein the marker support (10; 20) is provided with a support body (12, 26) being non-radioopaque, wherein several radioopaque marker elements (14, 14') are fixed to the support body (12; 26) and
wherein the nominal size S and/or the nominal distance D of the marker elements (14, 14') to each other is known,
image recording of the body part with a digital volume tomography device for generating a three-dimensional raw image of the body part,
measuring of the size S' and/or the distance D' of the marker elements (14) to each other in the raw image,
generating aberration values QS, QD by comparing the raw image size S' and/or the raw image distances D' with the respective nominal values S,D,
and
correction of the raw image using the aberration values QS, QD.

2. Method for generating a three-dimensional image of the body part according to claim 1, wherein the correction is the size correction of the raw image using the raw image distances D' in relation to the nominal distances D of the marker elements (14, 14').

3. Method for generating a three-dimensional image of a body part according to one of the preceding claims, wherein a quality indicator is issued, whose value results from the difference of the raw image size S' to the nominal size S of the marker elements (14, 14')

4. Method for generating a three-dimensional image of a body part according to one of the preceding claims, whereby at least ten marker elements (14, 14') are provided, wherein every marker element (14, 14') has a constant nominal distance of maximally 3.0 cm to its neighbor marker elements (14, 14'), and wherein several size corrections are performed locally omn the basis of the local comparison values.

5. Method for generating a three-dimensional image of a body part according to one of the preceding claims, wherein three neighbor marker elements (14, 14') respectively form an equilateral triangle.

6. Method for generating a three-dimensional image of a body part according to one of the preceding claims, wherein the marker elements (14) are arranged in at least two transversal planes.

7. Method for generating a three-dimensional image of a body part according to one of the preceding claims, wherein the radioopacity of the marker elements (14, 14') is different, with the following method step:
calibration of the brightness of the raw image on the basis of the known different radioopacity values of the marker elements (14, 14').

8. Method for generating a three-dimensional image of a body part according to one of the preceding claims, wherein the body part is the patient's jaw (22, 23), and the plurality of marker elements (14) are provided inside the dental arch (36, 37).

9. Method for generating a three-dimensional image of a body part according to one of the preceding claim, wherein at least one marker element (14') is provided outside the dental arch (36, 37).

10. Method for generating a three-dimensional Image of a body part according to claim 8 or 9, wherein the at least one marker element is provided outside of the patient's mouth.

## Revendications

1. Procédé de génération d'un enregistrement de tomographie volumétrique numérique tridimensionnelle radiologique d'une partie du corps d'un patient, notamment d'une tête d'un patient, avec les étapes de procédé suivantes:
fixer un support de marqueur (10; 20) sur ladite partie du corps, de préférence dans la bouche du patient, ledit support de marqueur (10; 20) comprenant un corps de support (12; 26) non radio-opaque sur lequel sont fixés plusieurs éléments marqueurs (14, 14') radio-opaques dont on sait la taille réelle S et/ou la distance réelle D mutuelle,
enregistrer ladite partie du corps avec un appareil de tomographie volumétrique numérique pour générer une image brute tridimensionnelle de ladite partie du corps,
mesurer la taille S' et/ou la distance D' mutuelle des éléments marqueurs (14) dans ladite image brute,
générer des valeurs d'écart QS, QD à partir d'une comparaison de la taille S' selon ladite image brute et/ou des distances D' selon ladite image brute avec les valeurs réelles respectives S, D, et
corriger ladite image brute sur la base des valeurs de comparaison QS, QD.

2. Procédé de génération d'un enregistrement tridimensionnelle d'une partie du corps selon la revendication 1, dans lequel la correction est une correction de taille dans l'image brute sur la base des distances D' selon ladite image brute par rapport aux distances réelles D des éléments marqueurs (14, 14').

3. Procédé de génération d'un enregistrement tridimensionnelle d'une partie du corps selon l'une quelconque des revendications précédentes, dans lequel un indicateur de qualité est délivré, dont la valeur résulte de l'écart de la taille S' dans l'image brute par rapport à la valeur réelle S des éléments marqueurs (14, 14').

4. Procédé de génération d'un enregistrement tridimensionnelle d'une partie du corps selon l'une quelconque des revendications précédentes, dans lequel au moins dix éléments marqueurs (14, 14'), chaque élément marqueur (14, 14') étant situé à une distance réelle constante d'au plus de 3,0 cm de ses éléments marqueurs voisins (14, 14'), et la correction de taille étant effectuée plusieurs fois localement sur la base des valeurs de comparaison locales.

5. Procédé de génération d'un enregistrement tridimensionnelle d'une partie du corps selon l'une quelconque des revendications précédentes, dans lequel trois éléments marqueurs (14, 14') voisins respectifs forment un triangle équilatéral.

6. Procédé de génération d'un enregistrement tridimensionnelle d'une partie du corps selon l'une quelconque des revendications précédentes, dans lequel les éléments marqueurs (14) sont disposés dans au moins deux plans transversaux.

7. Procédé de génération d'un enregistrement tridimensionnelle d'une partie du corps selon l'une quelconque des revendications précédentes, dans lequel la radio-opacité diffère entre les éléments marqueurs (14, 14'), avec l'étape de procédé suivante:
calibrer la luminosité de l'image brute sur la base des valeurs différentes connues de la radio-opacité des éléments marqueurs (14, 14').

8. Procédé de génération d'un enregistrement tridimensionnelle d'une partie du corps selon l'une quelconque des revendications précédentes, dans lequel est la mâchoire du patient (22, 23) et plusieurs éléments marqueurs (14) sont disposés à l'intérieur de l'arc de dents (36, 37).

9. Procédé de génération d'un enregistrement tridimensionnelle d'une partie du corps selon l'une quelconque des revendications précédentes, dans lequel au moins un élément marqueur (14') est disposé à l'extérieur de l'arc de dents (36, 37).

10. Procédé de génération d'un enregistrement tridimensionnelle d'une partie du corps selon les revendications 8 ou 9, dans lequel au moins un élément marqueur est disposé hors de la bouche du patient.
